(19) Europäisches Patentamt / European Patent Office / Office européen des brevets

(11) **EP 2 107 886 B1**

(12) **FASCICULE DE BREVET EUROPEEN**

(45) Date de publication et mention de la délivrance du brevet:
**01.05.2013 Bulletin 2013/18**

(21) Numéro de dépôt: **07872419.2**

(22) Date de dépôt: **20.12.2007**

(51) Int Cl.:
*A61B 5/03* (2006.01)

(86) Numéro de dépôt international:
**PCT/FR2007/002134**

(87) Numéro de publication internationale:
**WO 2008/096064 (14.08.2008 Gazette 2008/33)**

(54) **Détection non-invasive d'un paramètre électrique dépendant de la pression intralabyrinthique (PIL) chez un sujet**

Nichtinvasive Detektion von elektrischen Parametern, die vom Intralabyrinthdruck einer Person abhängen

Non invasive detection of an electrical parameter depending on the intralabyrinth pressure in a subject

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **08.01.2007 FR 0700078**

(43) Date de publication de la demande:
**14.10.2009 Bulletin 2009/42**

(73) Titulaire: **Université d'Auvergne Clermont 1
63000 Clermont-Ferrand (FR)**

(72) Inventeurs:
  • **AVAN, Paul, Alexandre, Louis**
    **F-63400 Chamalières (FR)**
  • **HASSOUN, Thierry, Claude**
    **F-63530 Volvic (FR)**

(74) Mandataire: **Colombet, Alain André et al
Cabinet Lavoix
62, rue de Bonnel
69448 Lyon Cedex 03 (FR)**

(56) Documents cités:
**WO-A-02/098291        US-A- 4 741 344
US-A- 5 601 091**

  • **HOEHMANN D ET AL: "THE HYDROPSFINDER: AN ELECTRONIC DEVICE FOR THE DIAGNOSIS OF ENDOLYMPHATIC HYDROPS" MEDICAL PROGRESS THROUGH TECHNOLOGY, SPRINGER VERLAG. BERLIN, DE, vol. 16, no. 4, 1 novembre 1990 (1990-11-01), pages 219-224, XP000229088 ISSN: 0047-6552**
  • **ERNST A ET AL: "Noninvasive assessment of the intralabyrinthine pressure. A new technique applied to patients with X-linked progressive mixed deafness syndrome with perilymphatic gusher during stapes surgery" ARCHIVES OF OTOLARYNGOLOGY HEAD AND NECK SURGERY, AMERICAN MEDICAL ASSOCIATION, US, vol. 121, no. 8, août 1995 (1995-08), pages 926-929, XP008082750 ISSN: 0886-4470**
  • **GERULL G ET AL: "LOW-FREQUENCY MASKING OF BRAINSTEM POTENTIALS" SCANDINAVIAN AUDIOLOGY, SCANDINAVIAN UNIVERSITY PRESS, OSLO, NO, vol. 20, no. 4, 1991, pages 227-234, XP008052797 ISSN: 0105-0397**

# Description

L'invention a trait à un procédé non invasif de mesure d'un paramètre électrique dépendant de la pression intralabyrinthique, chez un sujet, en particulier un potentiel microphonique cochléaire (PMC). Par sujet, on désigne ici un être humain ou un animal. L'invention concerne également un appareil permettant de mettre en oeuvre un tel procédé.

Dans la boîte crânienne d'un sujet, la fonction auditive est assurée par des organes en communication avec l'extérieur. Ces organes transmettent des vibrations sonores au système nerveux. Pour cela, les sons sont recueillis au niveau de l'oreille externe, par le conduit auditif et transmis, par l'intermédiaire du tympan, aux organes de l'oreille interne. Ces organes ne sont donc pas en contact direct avec l'extérieur. Ils sont isolés de l'extérieur par le tympan et se trouvent, normalement, à une pression interne correspondant globalement à la pression régnant à l'intérieur de la boîte crânienne.

Dans l'oreille interne les organes sensoriels responsables, d'une part, de l'audition et, d'autre part, de l'équilibre du sujet communiquent entre eux et baignent dans des liquides. Ces organes, à savoir le vestibule pour la fonction équilibre et la cochlée pour la fonction audition, forment ensemble le labyrinthe.

A l'intérieur de la cochlée, également appelée limaçon en raison de sa forme en spirale, se trouvent des cellules ciliées internes et des cellules ciliées externes disposées le long d'une membrane dénommée membrane basilaire.

La membrane basilaire vibre en fonction des sons reçus et les sépare selon les fréquences. Pour cela, la membrane présente deux parties distinctes. La partie située près de l'oreille moyenne est la plus mince et la plus tendue, elle collecte les sons aigus. L'autre partie est plus large et relâchée, elle collecte les sons graves.

Les sons transmis par la chaîne osseuse de l'oreille font vibrer les cils des cellules ciliées. Ces mouvements modifient les caractéristiques du milieu dans lequel se trouvent les cellules. En particulier, la pression dans la cochlée est affectée par les vibrations des cils. Ces variations de pression sont converties par l'organe de Corti en impulsions électriques transmises au cerveau par le nerf cochléaire. La cochlée fonctionne donc vis-à-vis des sons qu'elle reçoit comme un microphone puisqu'elle "traduit" les sons en impulsions électriques. Ce signal électrique correspond donc, de manière indirecte, à la pression régnant dans la cochlée. Le signal est affecté par la rigidité globale de l'oreille. Entres autres, la rigidité dépend de la pression intralabyrinthique : plus cette pression est élevée et plus la cochlée est rigide.

Le signal électrique émis par la cochlée est complexe. Il comprend essentiellement trois types de signaux électriques élémentaires.

Tout d'abord un potentiel électrique alternatif correspondant aux mouvements des cellules ciliées, essentiellement externes, en réponse aux vibrations de la membrane basilaire. Ce potentiel alternatif, dénommé couramment potentiel microphonique cochléaire uu "PMC", est directement représentatif du son transmis à l'oreille interne, autant au niveau de l'amplitude que de la phase et de la fréquence sonore.

Un deuxième type de signal élémentaire émis par la cochlée est un potentiel électrique continu dit potentiel de sommation ou "PS". Ce potentiel de sommation est lié aux mouvements des cellules ciliées, essentiellement internes. Il reproduit "l'enveloppe" de la stimulation acoustique reçue par la membrane basilaire. L'amplitude du potentiel continu est d'autant plus importante que la fréquence de la stimulation est haute (sons aigus) alors que le potentiel alternatif est plus important dans le signal complexe aux fréquences de stimulations basses (sons graves).

Un troisième type de signal élémentaire, nommé potentiel d'action composite ou "PAC", représente l'activité électrique des neurones et, en particulier, du nerf auditif. Il comprend, au début de la stimulation sonore, une onde négative suivie d'une onde positive, ces deux ondes se superposant en alternance de phases.

En d'autres termes, la réponse électrique des cellules ciliées de la cochlée à une stimulation acoustique présente trois composantes : une composante continue (PS) reproduisant l'enveloppe de la stimulation, une composante alternative (PMC), qui correspond à la fréquence du son pur et une composante composite (PAC) reflétant l'activité du système nerveux. Cette réponse électrique complexe est de l'ordre du microvolt.

Le rapport entre la composante continue (PS) et la composante alternative (PMC) dépend de la fréquence de la stimulation sonore.

L'ensemble de ces signaux forme une réponse cochléaire globale enregistrée habituellement par une technique invasive appelée électrocochléographie. Cette technique implique la mise en place d'une électrode le plus près possible de la cochlée, c'est-à-dire qu'elle nécessite le percement du tympan. Cette technique est donc difficile à mettre en oeuvre et elle est traumatisante pour le sujet.

On connaît par US-B-6 589 189 une méthode non invasive de mesure indirecte de la pression intracrânienne à partir des signaux acoustiques émis par la cochlée. Cette méthode est basée sur l'enregistrement de ces signaux réémis naturellement par la cochlée, en direction de l'oreille externe, lorsqu'elle est stimulée. Ces signaux, dénommés otoémissions acoustiques ou "OAE" reflètent l'activité des cellules ciliées externes. L'enregistrement se fait par un microphone inséré dans le conduit auditif et qui enregistre la réponse de la cochlée à des stimuli auditifs.

Dans cette méthode, on suppose que la pression à l'intérieur de la cochlée reflète la pression intracrânienne. En d'autres termes, on suppose dans US-B-6 589 189 que la pression est uniforme dans toute la boîte crânienne. Cette méthode se base sur l'activité des cellules ciliées externes, pour autant que ces dernières sont en état de fonctionner normalement. Autrement dit, ces cellules doivent être capables de générer une otoémission acoustique mesurable, de manière reproductible à chaque stimulation et pour chaque oreille. Ceci est avéré tant qu'il n'y a pas d'altération de l'audition chez le sujet. L'absence d'otoémissions acoustiques participe au dépistage, de manière non invasive et connue en ORL, de la surdité au niveau de la cochlée chez les sujets.

En revanche, il n'est pas possible d'appliquer cette méthode lorsque le signal acoustique a disparu, c'est-à-dire dans la plupart des cas où un défaut auditif est présent. En particulier, cette méthode n'est pas applicable sur des sujets présentant des troubles auditifs tels que la maladie de Menière.

Cette maladie se traduit par différents symptômes présents simultanément tels que des vertiges, des acouphènes et une surdité plus ou moins importante. Ces symptômes évoluent dans le temps, par cycle, le sujet étant soumis à des crises plus ou moins fréquentes. Une dégradation des conditions régnant au sein du labyrinthe, du fait que les organes de l'audition et de l'équilibre communiquent entre eux, induit l'apparition de ces symptômes.

La maladie de Menière, au stade final, se traduit par une surdité totale et la disparition des vertiges. Ceci correspond à une destruction totale des organes impliqués autant dans la fonction auditive que dans la fonction équilibre.

Plusieurs travaux (Schuknecht HF. Pathology of the ear, 1993. Lea and Febiger, Philadelphia, USA, 672pp. Kimura RS, Schucknecht HF, Membranous hydrops in the inner ear of the guinea pig after obliteration of the endolymphatic sac. Pract.Otorhinolaryngol. 1965, 27, 343-354. Hallpike CS and Cairns H, Observations on the pathology of Menière's syndrome. Proc.Roy.Soc.Med. 1938, 13, 1317-1331) ont montré que cette maladie pouvait être due à une dérégulation de la pression intralabyrinthique ou "PIL", celle-ci augmentant de façon importante et n'étant plus en corrélation avec la pression intracrânienne ou "PIC".

Une pression intralabyrinthique élevée, liée à la maladie de Menière, est avérée sur la base des données recueillies par autopsie de sujets où une distension considérable de certains compartiments intralabyrinthiques a été constatée. Une telle distension traduit une hydropie intralabyrinthique.

Cette dérégulation de la pression intralabyrinthique pourrait être due, d'après certains travaux (Horner, K.C., Old theme and new reflections : hearing impairment associated with endolymphatic hydrops. Hear.Res. 52 (1991) 147-156. Salt AN, DeMott J. Time course of endolymph volume increase in experimental hydrops measured in vivo with an ionic volume marker. Hear Res. 1994 Apr;74(1-2):165-72.Portmann M. Meniere's disease, Rev Laryngol Otol Rhinol (Bord). 1990;111(5):419-21), à un excès de sécrétion ou à un défaut de résorption du liquide intralabyrinthique.

Chez des sujets susceptibles d'être atteints de la maladie de Menière, la détection rapide d'une variation de la pression intralabyrinthique participe à l'appréciation d'un risque d'une crise, et éventuellement de son intensité, ce qui permet de prendre toutes dispositions pour éviter que les vertiges liés à ces crises aient des conséquences fâcheuses, par exemple qu'ils soient une cause d'accident par exemple d'accident de la route.

Il est donc intéressant de pouvoir mesurer un paramètre représentatif de la pression intralabyrinthique, et en particulier de détecter les variations de ce paramètre représentatif chez un sujet, notamment dans le cas d'un sujet atteint de la maladie de Menière, ce que ne permet pas la méthode décrite dans US-B-6589189.

On connaît par US-A-4 741 344 un procédé non invasif de collecte de signaux électriques émis par la cochlée en réponse à une stimulation auditive. La collecte des signaux s'effectue à l'aide d'une électrode, d'une configuration particulière, ayant subi un traitement de surface permettant une utilisation avec un gel. Une telle configuration de l'électrode permet d'améliorer la collecte du signal, compte tenu d'un bruit de fond important. Ce procédé ne permet pas néanmoins de s'affranchir des artefacts et d'isoler la réponse de la cochlée.

US-A-5 601 091 décrit un appareil de mesure audiométrique, de manière non invasive, par enregistrement de signaux en réponse à un stimulus. On enregistre un signal émis par le tronc cérébral et un signal émis par la cochlée, en l'espèce, une otoémission acoustique (OEA). Chaque signal est collecté par une électrode donnée. Les signaux recueillis permettent de réaliser de la tympanométrie et d'apprécier l'état de l'audition. La présence de bruits de fond ou artefacts affectent la collecte de signaux. Par ailleurs, cet appareil ne permet que de détecter la présence de fluide dans l'oreille moyenne mais pas de mesurer la pression intralabyrinthique.

C'est à ce problème qu'entend plus particulièrement répondre l'invention en proposant une méthode de détection d'un paramètre représentatif de la pression intralabyrinthique de manière non invasive, simple et rapide, chez un sujet atteint de la maladie de Menière, ainsi qu'un appareil permettant cette détection.

A cet effet, l'invention a pour objet un procédé non invasif de détection d'un paramètre électrique dépendant de la pression intralabyrinthique ou "PIL" chez un sujet soumis à une stimulation sonore répétitive dont l'origine temporelle et la fréquence sont prédéterminées, en collectant, à l'extérieur de la boîte crânienne du sujet, des signaux électriques émis par la cochlée en réponse à cette stimulation, procédé comprenant des étapes consistant à :

> a) envoyer vers la cochlée des stimulations sonores de type bouffées tonales à phases alternées,
> b) collecter les réponses électriques de la cochlée et du nerf auditif à ces stimulations,
> c) isoler la composante d'une réponse correspondant à la moyenne des réponses électriques à une stimulation à phase positive moins la moyenne des réponses à une stimulation à phase négative,

d) éliminer de cette réponse isolée les signaux dont l'origine temporelle est identique à l'origine temporelle de la stimulation et

e) ce grâce à quoi on obtient un potentiel microphonique cochléaire (PMC) type représentatif de la pression intralabyrinthique du sujet.

L'élimination de la partie de la réponse comportant le PMC, de signaux apparaissant dès l'origine de la stimulation permet de supprimer des artefacts et /ou des parasites, de manière sûre, rapide et totale du signal électrique alternatif émis par la cochlée.

On obtient ainsi une représentation fidèle de la propagation du son à l'intérieur de l'oreille. En cas de variation de la pression intralabyrinthique, la phase du son est affectée au cours de sa propagation, ce qui se traduit par une variation de la composante de la réponse cochléaire comportant le PMC.

Un tel procédé est applicable quel que soit l'état du patient, c'est-à-dire que celui-ci soit sain ou sourd, puisqu'il est indépendant de l'état des cellules ciliées pourvu qu'il en reste certaines. En effet, ce procédé est basé sur une caractéristique physique du milieu, à savoir la propagation des sons, et non pas sur une mesure d'un paramètre lié à l'activité du milieu.

De plus, il permet de s'affranchir des artéfacts tant d'origine biologique, c'est-à-dire dus au système neurologique et/ou musculaire, que d'origine extérieure, notamment ceux dus aux instruments électriques composant l'installation.

Selon des aspects avantageux mais non obligatoires de l'invention, un tel procédé peut incorporer une ou plusieurs des étapes suivantes :

- il comprend, après l'étape e) une étape de comparaison entre la réponse et celle obtenue lors d'une autre campagne de mesure, afin d'apprécier l'évolution du PMC.
- Lors de l'étape a) chaque bouffée tonale de la stimulation sonore a un spectre centré sur une fréquence de 1 kHz.
- Lors de l'étape a), la stimulation est répétée de 20 à 200 fois, l'origine temporelle étant connue.
- Lors de l'étape a), on effectue une stimulation à phase alternée à 180°, une fois sur deux, de manière à éliminer, de la réponse (R) électrique de la cochlée et du nerf auditif associée à cette stimulation, le potentiel microphonique cochléaire (PMC) et à obtenir une réponse ($R_1$) ne contenant que le potentiel de sommation (PS) et le potentiel d'action composite (PAC) pour en déduire ensuite, de la différence entre la réponse (R) reçue et la réponse (R1) reçue une fois sur deux, une valeur du potentiel microphonique cochléaire (PMC) global.
- Lors de l'étape e), on tient compte des réponses électriques apparaissant deux premières millisecondes de la stimulation.

L'invention concerne également un appareil de détection d'un paramètre électrique dépendant de la pression intralabyrinthique pour la mise en oeuvre d'un procédé selon l'une des caractéristiques précédentes, comprenant un module d'émission vers la cochlée d'une stimulation sonore répétitive, le module d'émission de la stimulation comprenant un tube acoustique, le tube acoustique ayant une longueur connue, reliant un dispositif de restitution du son, configuré pour être placé au voisinage du conduit auditif du sujet, à un haut-parleur, au moins deux capteurs d'un signal électrique émis par la cochlée en réponse, à la stimulation émise par le module d'émission, lesdits capteurs étant reliés à un module de collecte des données, relié à une unité de commande comprenant au moins une unité de mesure du temps propre à synchroniser une unité de conversion d'un signal provenant du module d'émission de la stimulation avec une unité de conversion d'un signal reçu par la module de collecte des données et comprenant par ailleurs une unité de traitement des données, l'unité de commande étant reliée à une unité de stockage des données.

Selon des aspects avantageux mais non obligatoires de l'invention, un tel appareil peut incorporer une ou plusieurs caractéristiques suivantes :

- Le tube a une longueur minimale de 30 cm.
- Les au moins deux capteurs comprennent trois électrodes, deux formant les bornes positive et négative et une autre formant la masse.
- Il comprend deux unités de mesure du temps, l'une pour le module d'émission de la stimulation, l'autre pour le module de collecte des réponses, synchronisées entre elles.

L'invention sera mieux comprise et d'autres avantages de celle-ci apparaîtront plus clairement à la lecture de la description qui va suivre, donnée à titre d'exemple et faite en se référant aux dessins annexés dans lesquels :

- la figure 1 est une représentation schématique d'une installation de mesure de la pression intralabyrinthique selon le procédé conforme à l'invention en place sur un sujet,
- la figure 2 est une courbe illustrant un potentiel microphonique cochléaire, isolé, avec la tension en ordonnée et le temps en abscisse et
- la figure 3 est une courbe similaire à la figure 2 d'un potentiel microphonique cochléaire non isolé, avec des artefacts.

Lors d'une stimulation sonore appliquée à un sujet, la réponse électrique de la cochlée, notée R, se présente sous la forme de signaux complexes correspondant aux différents potentiels d'origine cochléaire.

Cette réponse R correspond à différents potentiels électriques.

Pour rappel, le potentiel d'action composite ou "PAC", est d'une manière générale, le marqueur de l'activité synchrone du nerf auditif. Le potentiel de sommation ou "PS" correspond à une réponse continue des cellules ciliées lors de la stimulation et le potentiel microphonique ou "PMC" représente la réponse de type alternatif émise par les cellules ciliées externes en réponse à la stimulation. Ces paramètres sont liés par la relation :

$$R = PAC + PS + PMC.$$

Le potentiel microphonique ou "PMC" reproduit la stimulation sonore. En d'autres termes, sachant que la propagation d'un son est fonction des conditions physico-chimiques du milieu dans lequel il se propage, une variation du PMC est donc représentative de la variation des conditions physico-chimiques régnant dans le milieu de propagation du son, dans le labyrinthe. En effet, les organes de la cochlée baignent, comme les autres organes du labyrinthe, dans un liquide à une pression donnée dite pression intralabyrinthique ou "PIL".

Chez un sujet normalement entendant, la pression de la boîte crânienne et la pression intralabyrintique sont sensiblement les mêmes. En revanche, pour un sujet atteint de la maladie de Meniere, il en est tout autrement. Chez un tel sujet, il a été mis en évidence (Kimura RS, Schucknecht HF, Membranous hydrops in the inner ear of the guinea pig after obliteration of the endolymphatic sac. Pract.Otorhinolaryngol. 1965, 27, 343-354. Hallpike CS and Cairns H, Observations on the pathology of Menière's syndrome. Proc.Roy.Soc.Med. 1938, 13, 1317-1331.) une pression intralabyrinthique élevée et supérieure à celle des autres régions de la boîte crânienne.

L'une des difficultés pour mesurer la pression intralabyrinthique chez un sujet est due au fait que les différents compartiments du labyrinthe sont inclus dans l'os temporal et ne sont donc pas directement accessibles à des capteurs. On ne peut pas la mesurer directement et, qui plus est, par des méthodes non invasives.

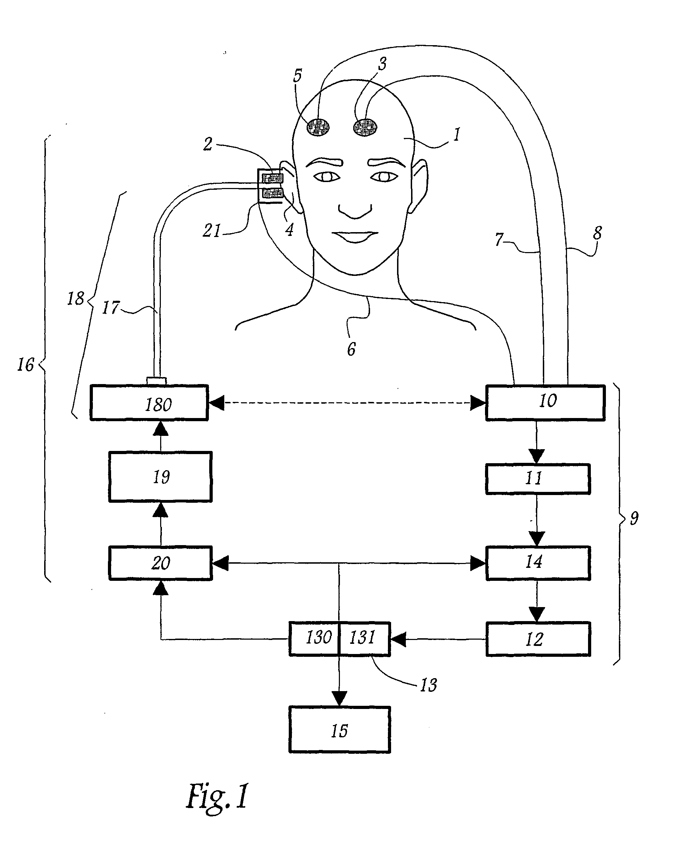

Pour effectuer une détection indirecte et non invasive de la PIL selon le procédé de l'invention, on utilise un appareil tel celui illustré à la figure 1.

Cette installation comprend des capteurs d'un signal électrique émis par la cochlée en réponse à un stimulus, ou électrodes, disposés au niveau de la tête 1 du sujet. On utilise au moins deux capteurs 2, 3 dont un, référencé 2, est positionné dans le conduit auditif externe 4, le plus près possible de l'oreille interne mais sans traumatisme pour le sujet, notamment sans percer le tympan.

Un autre capteur 3 est situé sur la tête 1 du sujet à une distance suffisante du premier capteur 2 pour enregistrer des différences de potentiel. Avantageusement, on place ce second capteur 3 au niveau du front, à une distance d'environ 15 centimètres du premier.

De manière préférée mais non obligatoire, on utilise un troisième capteur 5 disposé à un autre endroit de la tête 1 du sujet, par exemple près de la tempe. Ces trois capteurs vont constitués trois pôles de détection de la réponse électrique R. En l'espèce, celui placé près du conduit auditif forme une borne positive 2, celui sur le front une borne négative 3 et le troisième capteur 5 fait masse.

De préférence, on utilise comme capteurs, des électrodes connues en soi telles celles utilisées en électrocardiographie. De telles électrodes sont généralement autocollantes et aisément manipulables. Elles peuvent être mises en place par le sujet lui-même.

Pour améliorer le confort de ce dernier, l'électrode 2 située au niveau du conduit auditif peut être équipée d'un bouchon en mousse, percé d'un orifice central. Un tel bouchon en mousse est de préférence recouvert d'un matériau anallergique, lui-même bon conducteur électrique, par exemple une fine feuille d'or.

Ces trois électrodes 2, 3, 5 sont reliées par des liaisons filaires respectives 6, 7, 8 à un module 9 de collecte des données. De manière avantageuse, les différents fils 6, 7, 8 doivent avoir une longueur similaire, ne pas faire de boucle et/ou ne pas présenter de torsion, afin de ne pas générer des perturbations électromagnétiques.

Ce module 9 comprend avantageusement une unite de préamplification différentielle 10 permettant de convertir des signaux différentiels en un signal ou réponse R référencée. Sous cette forme, le signal est plus aisément manipulable c'est-à-dire que l'on peut aisément l'amplifier et lui appliquer des filtres analogiques ou numériques par des techniques connues en soi.

Une unité d'amplification 11 est disposée en aval de l'unité de préamplification 10 et en amont d'une unité 12 d'isolation.

Dans la mesure où les signaux recueillis sont de faible intensité, puisqu'ils ont généralement une tension de l'ordre du microvolt, l'isolation du signal de perturbations provenant d'autres unités de l'appareil est avantageusement faite par une unité d'isolation 12 numérique.

La réponse R ainsi amplifiée et/ou filtrée par l'unité d'amplification 11 est dirigée vers une unité 14 de conversion des données, avantageusement intercalée entre l'unité d'amplification 11 et l'unité d'isolation 12. Cette unité 14 est un convertisseur analogique-numérique. Une telle unité permet de convertir la réponse R en données numériques avec des résolutions et des fréquences données.

Cette unité 14 de conversion analogique-numérique permet d'effectuer un échantillonnage de la réponse dont le début d'émission doit être parfaitement connu et synchronisé par rapport au début de la stimulation sonore.

Pour cela, l'unité 14 de traitement est reliée à une unité 13 de commande. Cette unité 13 de commande comprend une unité 130 de traitement des données, par exemple un microcontrôleur et au moins une unité 131 de mesure du temps.

Les unités 131 de mesure du temps et 130 de traitement des données sont, avantageusement, situées de manière que la mesure du signal en aval de l'unité 12 par rapport à l'amplificateur 11 ne soit pas perturbée.

En variante, les données sont dirigées, avant ou après traitement, vers une unité 15 de stockage des données. Cette unité 15 comprend des supports connus en soi, par exemple, des supports numériques tels que des cartes mémoires, des cd-rom, des dvd-rom ou des supports analogiques par exemple, des impressions papier.

L'appareil comprend également un module de stimulation 16 de la cochlée du sujet. Il est à noter que, pour éviter toute perturbation électromagnétique, le module de stimulation 16 est éloigné du module 9 de collecte des signaux.

Ce module 16 comprend un tube acoustique 17 relié, à une extrémité, au capteur 2 inséré dans l'oreille du sujet et, à l'autre extrémité, à un haut parleur 180. Ici, le terme écouteur 18 désigne le haut parleur 180, le tube acoustique 17 et le dispositif de restitution du son 21 placé près de l'oreille du sujet. Dans l'exemple, le dispositif 21 est une oreillette permettant de restituer le son. Un côté de cette oreillette 21 est équipé d'une feuille d'or formant l'électrode 2. En d'autres termes, les dispositifs de restitution du stimulus et de collecte de la réponse sont confondus. Dans un mode de réalisation non illustré, ces dispositifs sont distincts. L'écouteur 18 est sous la commande d'un amplificateur audio 19 commandé par l'unité 13 de commande.

Entre l'amplificateur 19 et l'unité 13, on intercale une unité 20 de conversion numérique-analogique qui génère un son. Cette unité 20 de conversion de données permet de convertir un signal numérique provenant de l'unité 13 en un signal analogique propre à être amplifié par l'unité d'amplification 19.

Les unités 14 et 20 de conversion analogique-numérique et numérique-analogique sont, sous l'action de l'unité 131 de mesure du temps, parfaitement synchronisées. De cette manière, les origines temporelles de la stimulation sonore et de la réponse R reçue sont connues et identiques.

Un tel appareil est adapté pour avoir un encombrement suffisamment faible pour faciliter son transport et son utilisation par le sujet, de manière autonome. En particulier, l'alimentation en énergie est obtenue, selon un aspect de l'invention qui n'est pas illustré, par des batteries rechargeables ou des piles.

Cet appareil permet l'application du procédé non invasif de détection d'une variation de la pression intralabyrinthique, conforme à l'invention et décrit ci-après.

Lors d'une première étape a), on envoie vers la cochlée des stimulations sonores.

Le sujet, équipé des capteurs 2, 3, 5 tels que représenté à la figure 1, reçoit une stimulation sonore par l'intermédiaire de l'oreillette 21 placée à proximité de son conduit auditif externe 4.

Un tel dispositif 21 de restitution du son doit avoir un niveau de sortie maximale important, au moins de l'ordre de 125 dB, de manière à être alimenté avec une tension faible, de l'ordre de 10 à 20 millivolts, pour l'application envisagée. Ceci permet d'obtenir un niveau de sortie de la stimulation sonore de l'ordre de 70 à 75 dB au dessus du seuil auditif du sujet, ce qui est compatible avec les valeurs admissibles par l'oreille humaine.

Cette oreillette 21 est reliée au module de stimulation 16 par un tube 17 acoustique. Ce tube 17 a une longueur minimale, de manière que se produise un décalage temporel entre l'origine de la stimulation c'est-à-dire l'émission par le module de stimulation 16 d'un son, et la restitution par l'oreillette 21 de ce son. Ce décalage, basé sur la vitesse de propagation du son dans l'air, doit être de l'ordre de la milliseconde, ce qui implique une longueur minimale du tube acoustique 17 d'environ 30 centimètres.

Cette transmission du son entre le module de stimulation 16 et l'oreillette 21 placée sur l'oreille 4 du sujet est une transmission uniquement acoustique, en utilisant la vitesse de propagation du son dans l'air contenu dans un tube souple.

Le tube 17 a néanmoins un effet d'absorption du stimulus et donc d'atténuation du son perçu en sortie du dispositif 21 de restitution du son. Le matériau, la longueur et les caractéristiques physico-chimiques du tube 17 sont donc choisis de manière à ne générer un effet atténuateur du son que de l'ordre de 10 dB.

Compte tenu des faibles intensités de la réponse R enregistrée, il est important que la vibration mécanique de l'oreillette 21 placée dans l'oreille du sujet, généralement une oreillette de type prothèse auditive, soit minimale. Typiquement, il est nécessaire d'avoir une distorsion harmonique de l'oreillette 21 inférieure à 0,5 % aux fréquences utilisées, ces fréquences étant de l'ordre de 1 à 2 kHz.

De même, l'oreillette doit présenter une courbe de réponse globalement plate pour des fréquences comprises entre 0, 5 et 3 kHz.

La stimulation sonore émise par le module de stimulation 16 est contrôlée et connue. Le stimulus sonore envoyé a une fréquence, une amplitude, et une phase connues.

Ce stimulus est formé à partir d'une onde de base sinusoïdale. En effet, on veut produire un stimulus sonore de niveau calibré capable d'atteindre 80 à 90 décibels au dessus du seuil auditif normal, cela pour une tension efficace appliquée au niveau de l'oreillette 21 d'une centaine de millivolts au maximum. Une telle tension permet de minimiser les signaux parasites d'origine électrique, éventuellement rayonnés par l'oreillette. Ces signaux parasites seraient en effet captés, compte tenu de leurs caractéristiques et de leurs valeurs, par les électrodes 2, 3, 5 disposées sur le sujet. Un niveau de stimulus de 90 décibels permet, chez les sujets ayant une baisse importante de l'audition, d'obtenir une réponse tout en respectant les maxima tolérables par l'oreille.

Le stimulus, c'est-à-dire en l'occurrence chaque onde sonore, a un spectre centré sur une fréquence de 1 kilohertz. Des travaux (Avan, P., Büki, B., Maat, B., Dordain, M. and Wit, H.P., Middle-ear influence on otoacoustic emissions. I: Noninvasive investigation of the human transmission apparatus and comparison with model results, Hear.Res. 140 (2000), 189-201 et de Buki B, Chomicki A, Dordain M, Lemaire JJ, Wit HP, Chazal J, Avan P. Middle-ear influence on otoacoustic emissions. II: contributions of posture and intracranial pressure. Hear Res. 2000 Feb;140(1-2): 202-11.) ont montré que c'est à des fréquences voisines de cette valeur qu'un effet maximum de la pression intralabyrinthique sur la phase de la réponse émise par la cochlée est observé.

Dans un autre mode de réalisation non illustré, chaque bouffée tonale a un spectre centré sur une fréquence différente, par exemple une fréquence de 2 kHz.

De préférence, on n'utilise pas un son isolé comme stimulus mais un train de stimuli, c'est-à-dire un train d'ondes sonores dont l'origine temporelle est précise et déterminée grâce à l'unité 131.

Ce train de stimuli est réalisé à partir de sinusoïdes d'origine temporelle connue. Cette origine sert également d'origine temporelle pour le traitement de la réponse R c'est-à-dire des signaux recueillis par les électrodes 2, 3, 5. Pour cela, la synchronisation entre les unités 20 et 14 doit être précise. Avantageusement, les unités 20 et 14 sont soumises au contrôle d'une même unité 13. Dans un mode de réalisation non illustré, deux unités 131 de mesure du temps fonctionnant en parallèle et synchronisées du temps sont utilisées.

Pour éviter une attaque trop brutale du train de stimuli sur l'écouteur, une "enveloppe" sonore est appliquée aux stimuli. Cette enveloppe a une forme de cosinus carré de brève durée. Elle est appliquée préalablement à l'envoi des ondes sinusoïdales stimulantes qui, elles, sont émises à pleine amplitude.

Au bout d'un certain temps préalablement choisi par l'utilisateur, c'est-à-dire soit le sujet lui-même soit un tiers, le train de stimuli revient à zéro par l'intermédiaire également d'une enveloppe sonore en forme de cosinus carré, symétrique et opposée à la première. Le temps de plateau, c'est-à-dire le temps où l'on envoie les stimuli, est globalement compris entre 10 et 80 millisecondes.

Ce type de stimulation est généralement connu sous le nom de bouffée tonale ou tone-burst à phases positive et négative alternées. Il s'agit en fait d'envoyer une série discontinue d'ondes avec un départ et une fin progressive. Lors de l'étape a) la stimulation envoyée est donc de type bouffée tonale à phases alternatives.

Il est nécessaire d'envoyer un tel stimulus plutôt qu'une stimulation sonore continue pour différentes raisons. Une raison est que, comme indiqué précédemment, le signal que l'on cherche à collecter dans la réponse R, en l'espèce le PMC, est très faible et qu'il est généralement noyé dans un bruit de fond formant un signal électrique souvent dix fois plus important. En d'autres termes le signal recherché, de l'ordre du microvolt, est noyé dans un bruit de fond de l'ordre du centième de millivolt.

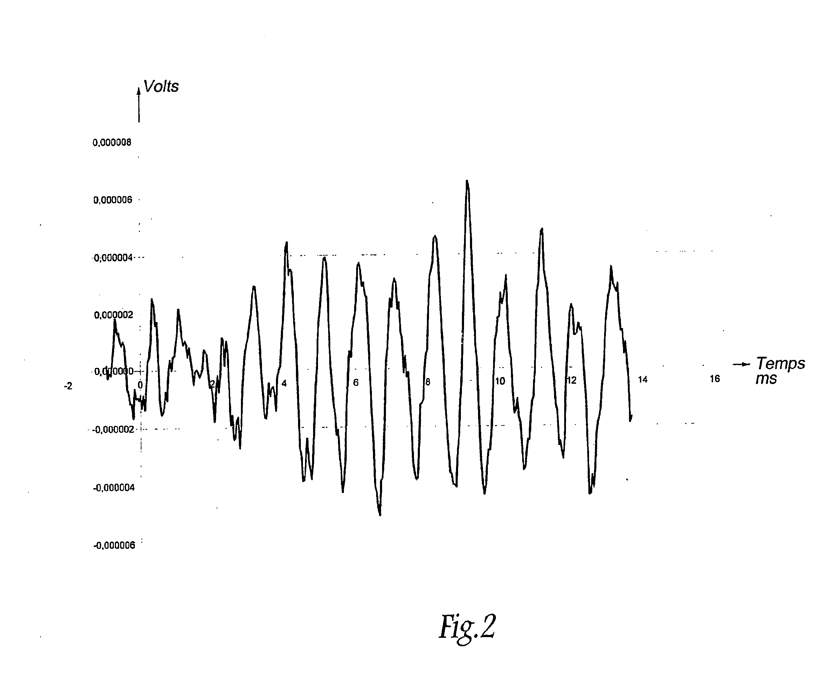

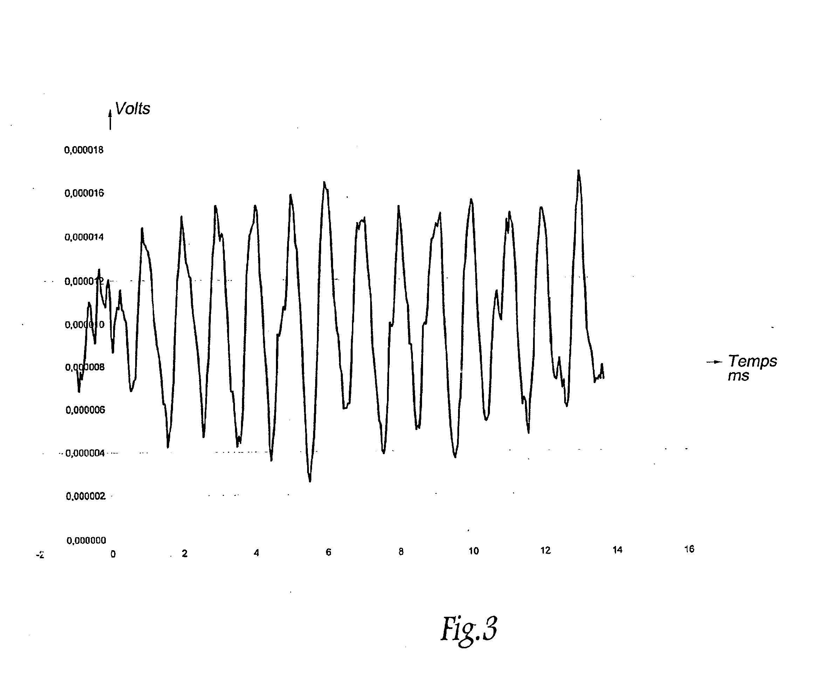

Ce bruit de fond général comprend une composante d'origine biologique, c'est-à-dire provenant du système nerveux et/ou musculaire. Cette composante est un bruit, par définition, aléatoire et non répétitif. C'est-à-dire que l'on ne peut pas le reproduire et que son intensité n'est pas connue. Son intensité reste toutefois modérée par rapport au PMC, bien que généralement supérieure au PMC. Cette composante biologique est indépendante du stimulus. Elle est mise en évidence aux figures 2 et 3 où un signal électrique est enregistré avant le début de la stimulation. Il s'agit de la partie des courbes située à gauche de l'axe des ordonnées.

Si l'on répète ce stimulus à l'identique c'est-à-dire si l'on envoie une série de bouffées tonales plusieurs fois de suite, typiquement entre 20 à 200 fois, on émet des bouffées tonales qui sont toujours identiques à intervalles réguliers et donc aisément repérables.

Lors d'une deuxième (étape b), on collecte les réponses R électriques de la cochlée et du nerf auditif à ces stimulations. Le traitement par moyennage des réponses, de manière synchrone, à l'émission du train de stimuli permet de différencier et d'extraire dans la réponse complexe reçue, un signal représentatif de la stimulation reçue, c'est-à-dire d'isoler le potentiel microphonique cochléaire (PMC). En d'autres termes, lors d'une troisième étape c), on isole la composante d'une réponse correspondant à la moyenne des réponses électriques à une stimulation positive moins la moyenne des réponses à une stimulation négative.

Le PMC est une réponse représentative du stimulus. En d'autres termes, en envoyant ainsi un train de stimuli sous forme de bouffées tonales dont on connaît l'origine on sait que le PMC va être émis proportionnellement au stimulus, à des instants connus et réguliers. Ainsi, il est aisément repérable par rapport au bruit de fond d'origine biologique.

Néanmoins, il existe une deuxième composante parasite du bruit de fond. Cette deuxième composante est suffisamment forte pour masquer le PMC. Cette composante est, elle, non aléatoire et en général, parfaitement connue. Il s'agit du bruit électrique transmis par l'écouteur 18 lui-même, en fait un signal parasite électrique rayonné.

Ce deuxième bruit a une forme similaire à celle du stimulus émis. En d'autres termes, ce bruit parasite est répétitif et il est émis avec une intensité et une fréquence tout à fait comparables à celle du PMC puisqu'il est, lui aussi, lié au stimulus. Il est donc, a priori, difficile de pouvoir extraire le PMC seul, sans le bruit généré par l'installation.

Pour cela pendant longtemps les enregistrements de PMC ont été considérés comme étant peu intéressants et peu fiables puisqu'ils étaient facilement parasitables par les appareils de mesure, le bruit parasite étant suffisamment important pour masquer le PMC, avec le risque supplémentaire d'être pris pour le PMC par un opérateur insuffisamment averti.

De ce fait, lors de ce type d'enregistrement, il est connu d'éliminer volontairement le bruit parasite et le PMC de la réponse collectée en effectuant une stimulation à phases opposées. Une fois sur deux le stimulus est envoyé avec une phase φ une première fois et une phase φ + π la fois d'après. Ainsi, le signal reçu est annulé et, si le signal parasite est bien supprimé, le PMC est également supprimé. Dans ce cas, la réponse R₁ ne comprend plus que le potentiel de sommation PS et le potentiel d'action composite PAC selon la relation

$$R_1 = PS + PAC$$

Autrement dit, par différence entre la réponse R reçue et la réponse R₁ reçue avec une stimulation à phase alternée à 180° une fois sur deux, on déduit une valeur globale du potentiel microphonique cochléaire ou PMC. Il convient alors de "nettoyer" ce PMC de tout artéfact et parasite d'origine électrique.

Lors d'une quatrième étape d), on élimine de cette réponse isolée les signaux dont l'origine temporelle est identique à l'origine temporelle de la stimulation.

Pour cela, on utilise le fait que le signal parasite rayonné est d'origine électromagnétique à l'inverse du PMC qui, lui, est d'origine acoustique.

Ce signal parasite est en effet transmis par l'écouteur 18 relié au module de stimulation 16 dès la mise en marche du module 16 de stimulation.

Ce signal parasite apparaît donc dans la réponse R collectée des le début de l'émission du stimulus, puisqu'il est émis directement par les appareils électriques de l'installation dès leur mise en marche.

En d'autres termes, la vitesse de propagation du signal parasite n'est pas comparable à la vitesse de propagation du signal émis par la cochlée. Dans un cas, celui du signal parasite, on a une propagation d'une onde électrique c'est-à-dire quasi instantanément, dans l'autre cas, celui du signal émis par la cochlée, c'est la propagation d'un son dans l'air puis dans l'organisme qui est à prendre en compte.

C'est-à-dire que si l'on a un tube acoustique 17 d'environ 30 centimètres de long, le temps de propagation du son entre le module 16 et l'oreillette 21 est d'environ une milliseconde. Ce son reçu par le dispositif 21 de restitution du son est émis par ce dernier, passe dans l'oreille du sujet, stimule les cellules ciliées qui vibrent et répondent à ce son sous forme de signal électrique (PMC + PS) qui est alors détecté par les électrodes 2, 3, 5.

Des travaux (Zwislocki JJ. Some current concepts of cochlear mechanics. Audiology. 1983;22(6):517-29) ont montré que le déphasage entre la stimulation émise par l'oreillette 21 et sa réception par la cochlée, dit déphasage aller, résulte du temps mis pour que le son se propage à travers la structure de l'oreille, qui est connu pour être d'environ une milliseconde (Békésy G. von. Direct observation of the vibrations of the cochlear partition under a microscope. Acta Otolaryngol. 1952 Jun;42(3):197-201).

En d'autres termes, entre l'instant où la stimulation est émise par le module 16 de stimulation et l'instant où la réponse électrique émise par la cochlée est collectée par les électrodes 2, 3 et 5, il s'écoule environ 2 millisecondes.

Le parasite d'origine électromagnétique est, lui, rayonné tout de suite par le haut parleur 180, c'est-à-dire avant la première milliseconde et il est détecté par les électrodes quasi simultanément.

L'oscillation électrique émise en réponse au stimulus par la cochlée, c'est-à-dire le PMC, ne peut être détectée qu'au minimum deux millisecondes après le début de la stimulation et, en aucun cas, il ne peut y avoir émission d'une oscillation de la part de la cochlée avant la première milliseconde. En effet, avant cette première milliseconde la cochlée ne peut pas avoir reçu de stimulation, le stimulus étant encore en propagation dans le tube 17 en amont de l'oreillette 21.

Compte tenu de la longueur du tube 17 et de la vitesse de propagation du son dans l'air, il est en effet impossible que cette stimulation soit transmise par l'oreillette 21 avant une milliseconde.

Toute réponse reçue pendant cette première milliseconde ne peut donc avoir qu'une origine autre que la cochlée, c'est-à-dire être due à un parasite d'ordre électromagnétique. Une réponse R reçue pendant ces deux premières millisecondes traduit un parasitage d'origine électrique rayonné. La figure 3 illustre une réponse de ce type. L'amplitude crête à crête de la réponse atteint 10 microvolts, dès la première milliseconde, ce qui correspond sensiblement à la valeur maximale de la réponse.

Autrement dit, l'absence totale de réponse R autour de la fréquence correspondant à celle du stimulus, généralement voisine de 1 kHz, pendant les deux premières millisecondes garantit qu'il n'y a pas de parasite électrique rayonné.

Afin de quantifier ce signal parasite, on effectue une stimulation avec une fréquence qui varie entre deux valeurs. La propagation du stimulus induit une variation de phase du signal reçu en fonction de la fréquence. Le signal parasite d'origine électrique a lui une phase fixe comprise entre ces deux fréquences. Le PMC a une phase différente selon la fréquence de la stimulation. La variation de la phase avec la fréquence est sensiblement égale à 2π.τ où τ est le délai de propagation du signal. Pour le signal parasite pour lequel τ est nul, la propagation étant immédiate, la phase ne dépend pas de la fréquence. Une telle stimulation à fréquence variable est connue sous le nom de "stimulation sweep" ou "chirp". Il s'agit ni plus ni moins que d'une stimulation sonore dont la fréquence de la sinusoïde est modulée.

De cette manière, on quantifie et on extrait l'artéfact du PMC global afin de n'obtenir que le PMC émis par la cochlée. La figure 2 illustre une réponse de ce type. Le maximum de la réponse est d'environ 0,6 microvolt, une réponse voisine de 0,4 microvolt n'étant enregistrée qu'au bout de 4 millisecondes après le début de la stimulation. On remarque que, jusqu'à 2 millisecondes, l'intensité enregistrée correspond à l'intensité enregistrée avant la stimulation, c'est-à-dire l'enregistrement d'un bruit de fond biologique. Le signal représentatif de la réponse de la cochlée n'atteint sa pleine amplitude qu'à partir d'environ 3 millisecondes après le début de la stimulation.

Le PMC typique, sans parasitage, émis par la cochlée correspond à la transmission du son dans l'oreille c'est-à-dire à travers un milieu donné. Si le milieu dans lequel se propage le son subit une variation de pression, la phase est affectée lors de la propagation et donc le PMC, qui est un paramètre électrique, est lui aussi affecté. Le PMC typique traduit donc une valeur de la pression intralabyrinthique PIL

Dans une cinquième étape e), on considère la réponse ainsi obtenue comme étant un potentiel microphonique cochléaire type représentatif de la PIL.

Des essais réalisés chez l'animal, avec une mesure directe, invasive, de la pression en parallèle avec la mesure non invasive du PMC selon le procédé décrit ici, ont montré qu'une variation, même rapide, de la PIL peut être détectée tout aussi rapidement, en comparant les PMC entre deux campagnes de mesure. Il est à noter que, pour un même patient, les mêmes conditions de mesure donnent normalement les mêmes indications relatives à la PIL. Il y a donc reproductibilité de la méthode. Ces PMC étant débarrassés de tous signaux parasites de la manière décrite précédemment, ils sont représentatifs de la PIL et on peut apprécier si un changement de la PIL a eu lieu lors d'une sixième étape de comparaison des réponses obtenues lors de deux campagnes de mesure.

Cette détection indirecte de la PIL est mise en oeuvre pour un sujet atteint de la maladie de Menière quel que soit le stade de la maladie, pour autant que le sujet ne soit pas sourd profond. Dans ce cas, il s'agit du stade ultime de la maladie, le sujet n'ayant plus ni vertige, ni audition.

Un tel procédé, aisé à mettre en oeuvre, simple et rapide permet au sujet de détecter régulièrement l'évolution d'un paramètre électrique dépendant de la PIL. Tout changement de ce paramètre indique, potentiellement, un changement de la PIL. Ce changement est un élément à prendre en compte dans la détection de l'apparition des vertiges et la prévention de leur conséquence.

Une telle méthode, pour autant que le sujet n'est pas sourd, est applicable à un sujet sain ou pour le moins non atteint de la maladie de Menière.

## Revendications

1. Procédé non invasif de détection d'un paramètre électrique dépendant de la pression intralabyrinthique (PIL) chez un sujet soumis à une stimulation sonore répétitive dont l'origine temporelle et la fréquence sont prédéterminées, en collectant, à l'extérieur de la boîte crânienne du sujet, des signaux électriques émis par la cochlée en réponse à cette stimulation, ce procédé comprenant des étapes consistant à :

   a) envoyer vers la cochlée des stimulations sonores de type bouffées tonales à phases alternées,
   b) collecter les réponses électriques R de la cochlée et du nerf auditif à ces stimulations,
   c) isoler la composante d'une réponse correspondant à la moyenne des réponses électriques à une stimulation à phase positive moins la moyenne des réponses à une stimulation à phase négative,
   d) éliminer de cette réponse isolée les signaux dont l'origine temporelle est identique à l'origine temporelle de la stimulation et
   e) ce grâce à quoi on obtient un potentiel microphonique cochléaire type représentatif de la pression intralabyrinthique du sujet.

2. Procédé selon la revendication 1, **caractérisé en ce qu'**il comprend, après l'étape e) une étape de comparaison entre la réponse et celle obtenue lors d'une autre campagne de mesure, afin d'apprécier une évolution du PMC.

3. Procédé selon l'une des revendications précédentes, **caractérisé en ce que** lors de l'étape a) chaque bouffée tonale de ladite stimulation sonore a un spectre centré sur une fréquence de 1 Khz.

4. Procédé selon l'une des revendications précédentes, **caractérisé en ce que** lors de l'étape a), la stimulation est répétée de 20 à 200 fois, l'origine temporelle étant connue.

5. Procédé selon l'une des revendications précédentes, **caractérisé en ce que** lors de l'étape a), on effectue une stimulation à phase alternée à 180°, une fois sur deux, de manière à éliminer, de la réponse R électrique de la cochlée et du nerf auditif associée à cette stimulation, le potentiel microphonique cochléaire PMC et à obtenir une réponse $R_1$ ne contenant que le potentiel de sommation PS et le potentiel d'action composite PAC pour en déduire ensuite, de la différence entre la réponse R reçue et la réponse R1 reçue une fois sur deux, une valeur du potentiel microphonique cochléaire PMC global.

**6.** Procédé selon l'une des revendications précédentes, **caractérisé en ce que** lors de l'étape e), on tient compte des réponses électriques apparaissant en dehors des deux premières millisecondes de la stimulation.

**7.** Appareil de détection d'un paramètre électrique dépendant de la pression intralabyrinthique pour la mise en oeuvre d'un procédé selon l'une des revendications 1 à 6, comprenant un module (16) d'émission vers la cochlée d'une stimulation sonore répétitive, le module (16) d'émission de la stimulation comprenant un tube acoustique (17), le tube acoustique (17) ayant une longueur connue, reliant un dispositif (21) de restitution du son, configuré pour être placé au voisinage du conduit auditif du sujet, à un haut-parleur (180), au moins deux capteurs (2, 3, 5) d'un signal électrique émis par la cochlée en réponse, à la stimulation émise par le module (16) d'émission, lesdits capteurs (2, 3, 5) étant reliés à un module (9) de collecte des données, relié à une unité (13) de commande comprenant au moins une unité (131) de mesure du temps propre à synchroniser une unité (20) de conversion d'un signal provenant du module (16) d'émission de la stimulation avec une unité (14) de conversion d'un signal reçu par la module (9) de collecte des données et comprenant par ailleurs une unité (130) de traitement des données, l'unité de commande étant reliée à une unité (15) de stockage des données.

**8.** Appareil selon la revendication 7, **caractérisé en ce que** ledit tube (17) a une longueur minimale de 30 cm.

**9.** Appareil selon la revendication 7, **caractérisé en ce que** lesdit au moins deux capteurs comprennent trois électrodes (2 3, 5), deux formant les bornes positive (2) et négative (3) et une autre formant la masse (5).

**10.** Appareil selon la revendication 7, **caractérisé en ce qu'**il comprend deux unités (131) de mesure du temps, l'une pour le module (16) d'émission de la stimulation, l'autre pour le module (9) de collecte des réponses, synchronisées entre elles.

**Claims**

**1.** A non-invasive method of detecting an electrical parameter dependent on the intralabyrinthine pressure ILP in a subject subjected to a repetitive sound stimulation, the time origin and frequency of which are predetermined, by collecting, outside the cranial cavity of the subject, electrical signals emitted by the cochlea in response to this stimulation, this method comprising steps consisting in :

a) sending to the cochlea sound stimulations of the tone burst type with alternating phases,
b) collecting the electrical responses R from the cochlea and from the auditory nerve to these stimulations,
c) isolating the component of a response that corresponds to the average of the electrical responses to a positive phase stimulation minus the average of the responses to a negative phase stimulation,
d) eliminating from this isolated response the signals whose time origin is identical to the time origin of the stimulation and
e) thus obtaining a cochlear microphonic potential CMP of a type representative of the intralabyrinthine pressure of the subject.

**2.** The method as claimed in claim 1, **characterized in that** it comprises, after the step e), a step of comparison between the response and that obtained in another series of measurements, in order to evaluate a trend of the CMP.

**3.** The method as claimed in claim 1, **characterized in that**, in the step a), each tone burst of said sound stimulation has a spectrum centered on a frequency of 1 kHz.

**4.** The method as claimed in claim 1, **characterized in that**, in the step a), the stimulation is repeated 20 to 200 times, the time origin being known.

**5.** The method as claimed in claim 1, **characterized in that**, in the step a), a stimulation is effected with phase alternating by 180° once every two times so as to eliminate, from the electrical response R from the cochlea and from the auditory nerve associated to said stimulation, the cochlear microphonic potential CMP and to obtain a response $R_1$ containing only the summation potential SP and the composite action potential CAP in order toobtain, from the difference between the response R received and the response $R_1$ received once every two times, a value of the overall cochlear microphonic potential CMP.

**6.** The method as claimed in claim 1, **characterized in that**, in the step e), the responses appearing out of the two first milliseconds of the stimulation are taken into account.

**7.** An appliance for detecting an electrical parameter dependent on the intralabyrinthine pressure to implement a method as claimed in claim 1, comprising a module (16) emitting to the cochlea a repetitive sound stimulation, the stimulation emission module (16) comprising an acoustic tube (17), the acoustic tube (17) having a known length, linking a sound playback device (21), configured to be placed in the vicinity of the antrum auris of the subject, to a loudspeaker (180), at least two sensors (2, 3, 5) for an electrical signal emitted by the cochlea in response to the stimulation emitted by the emission module (16), said sensors (2, 3, 5) being linked to a data collection module (9), linked to a control unit (13) comprising at least one time measuring unit (131) for synchronizing a unit (20) for converting a signal originating from the stimulation emission module (16) with a unit (14) for converting a signal received by the data collection module (9), and comprising also a data processing unit (130), the control unit being linked to a data storage unit (15) .

**8.** The appliance as claimed in claim 7, **characterized in that** said tube (17) has a minimum length of 30 cm.

**9.** The appliance as claimed in claim 7, **characterized in that** said at least two sensors comprise three electrodes (2, 3, 5), two forming the positive (2) and negative (3) terminals and another forming the ground (5) .

**10.** The appliance as claimed in claim 7, **characterized in that** it comprises two time measuring units (131), one for the stimulation emission module (16), the other for the response collection module (9), synchronized with each other.

**Patentansprüche**

**1.** Nicht invasives Verfahren zur Ermittlung eines elektrischen Parameters, der vom intralabyrinthären Druck PIL bei einer Person abhängt, die einer wiederholten akustischen Stimulation unterzogen wird, deren zeitlicher Ursprung und Frequenz vorbestimmt sind, indem außerhalb der Schädelhöhle der Person elektrische Signale erfasst werden, die durch die Cochlea als Reaktion auf diese Stimulation ausgestrahlt werden, wobei dieses Verfahren Schritte umfasst, die aus Folgendem bestehen:

a) Senden akustischer Stimulationen des Typs Tonimpulse mit wechselnden Phasen in Richtung der Cochlea,
b) Sammeln der elektrischen Reaktionen R der Cochlea und des Hörnervs auf diese Stimulationen,
c) Isolieren der Komponente einer Reaktion, die dem Mittel der elektrischen Reaktionen auf eine Stimulation mit positiver Phase minus dem Mittel der Reaktionen auf eine Stimulation mit negativer Phase entspricht,
d) Beseitigen der Signale, deren zeitlicher Ursprung identisch mit dem zeitlichen Ursprung der Stimulation ist, von dieser isolierten Reaktion, und

e) dadurch Erhalten eines cochleären Mikrophonpotentials des Typs, der für den intralabyrinthären Druck der Person charakteristisch ist.

**2.** Verfahren nach Anspruch 1, **dadurch gekennzeichnet, dass** es nach dem Schritt e) einen Schritt des Vergleichens zwischen der Reaktion und derjenigen umfasst, die während einer anderen Messreihe erhalten wurde, um eine Entwicklung des cochleären Mikrophonpotentials PMC einzuschätzen.

**3.** Verfahren nach einem der vorhergehenden Ansprüche, **dadurch gekennzeichnet, dass** im Schritt a) jeder Tonimpuls der akustischen Stimulation ein Spektrum aufweist, das auf eine Frequenz von 1 Khz zentriert ist.

**4.** Verfahren nach einem der vorhergehenden Ansprüche, **dadurch gekennzeichnet, dass** die Stimulation im Schritt a) zwischen 20 und 200 Mal wiederholt wird, wobei der zeitliche Ursprung bekannt ist.

**5.** Verfahren nach einem der vorhergehenden Ansprüche, **dadurch gekennzeichnet, dass** im Schritt a) jedes zweite Mal eine Stimulation mit um 180° wechselnder Phase durchgeführt wird, derart, dass das cochleäre Mikrophonpotential PMC von der dieser Stimulation zugehörigen elektrischen Reaktion R der Cochlea und des Hörnervs beseitigt wird und eine Reaktion $R_1$ erhalten wird, die lediglich das Summationspotential PS und das Summenaktionspotential PAC enthält, um davon von der Differenz zwischen der empfangenen Reaktion R und der jedes zweite Mal empfangenen Reaktion $R_1$ einen Wert des gesamten cochleären Mikrophonpotentials PMC abzuleiten.

**6.** Verfahren nach einem der vorhergehenden Ansprüche, **dadurch gekennzeichnet, dass** im Schritt e) die elektrischen Reaktionen berücksichtigt werden, die außerhalb der zwei ersten Millisekunden der Stimulation auftreten.

**7.** Vorrichtung zur Ermittlung eines elektrischen Parameters, der vom intralabyrinthären Druck abhängt, zur Durchführung eines Verfahrens nach einem der Ansprüche 1 bis 6, die ein Modul zum (16) Ausstrahlen einer wiederholten akustischen Stimulation in Richtung der Cochlea umfasst, wobei das Modul (16) zum Ausstrahlen der Stimulation einen Hörschlauch (17) umfasst, wobei der Hörschlauch (17) eine bekannte Länge aufweist, eine Vorrichtung (21) zur Klangwiedergabe, die gestaltet ist, um in der Nähe des Gehörgangs der Person untergebracht zu werden, mit einem Lautsprecher (180) verbindet, mindestens zwei Sensoren (2, 3, 5) für ein als Reaktion auf die durch das Ausstrahlungsmodul (16) ausgestrahlte Stimulation durch die Cochlea ausgestrahltes elektrisches Signal, wobei die Sensoren (2, 3, 5) mit einem Modul (9) zur Erfassung der Daten verbunden sind, das mit einer Steuereinheit (13) verbunden ist, die mindestens eine Zeitmesseinheit (131) umfasst, die geeignet ist, eine Einheit (20) zur Umwandlung eines Signals, das von dem Modul (16) zum Aussenden der Stimulation stammt, mit einer Einheit (14) zur Umwandlung eines von dem Modul (9) zur Erfassung der Daten empfangenen Signals zu synchronisieren, und ferner eine Einheit (130) zur Verarbeitung der Daten umfasst, wobei die Steuereinheit mit einer Einheit (15) zur Speicherung der Daten verbunden ist.

8. Vorrichtung nach Anspruch 7, **dadurch gekennzeichnet, dass** der Schlauch (17) eine Mindestlänge vom 30 cm aufweist.

9. Vorrichtung nach Anspruch 7, **dadurch gekennzeichnet, dass** die mindestens zwei Sensoren drei Elektroden (2, 3, 5), zwei, die den positiven (2) und den negativen Anschluss (3) bilden, und eine, die die Masse (5) bildet, umfassen.

10. Vorrichtung nach Anspruch 7, **dadurch gekennzeichnet, dass** sie zwei Zeitmesseinheiten (131), eine für das Modul (16) zum Ausstrahlen der Stimulation und die andere für das Modul (9) zur Erfassung der Reaktionen, umfasst, die miteinander synchronisiert sind.

Fig.1

Fig.2

*Fig.3*

EP 2 107 886 B1

## RÉFÉRENCES CITÉES DANS LA DESCRIPTION

**Documents brevets cités dans la description**

- US 6589189 B
- US 4741344 A
- US 5601091 A

**Littérature non-brevet citée dans la description**

- **SCHUKNECHT HF.** Pathology of the ear. Lea and Febiger, 1993, 672
- **KIMURA RS ; SCHUCKNECHT HF.** Membranous hydrops in the inner ear of the guinea pig after obliteration of the endolymphatic sac. *Pract.Otorhinolaryngol.,* 1965, vol. 27, 343-354
- **HALLPIKE CS ; CAIRNS H.** Observations on the pathology of Menière's syndrome. *Proc.Roy.Soc.Med.,* 1938, vol. 13, 1317-1331
- **HORNER, K.C.** Old theme and new reflections : hearing impairment associated with endolymphatic hydrops. *Hear.Res.,* 1991, vol. 52, 147-156
- **SALT AN ; DEMOTT J.** Time course of endolymph volume increase in experimental hydrops measured in vivo with an ionic volume marker. *Hear Res.,* Avril 1994, vol. 74 (1-2), 165-72
- **PORTMANN M.** Meniere's disease. *Rev Laryngol Otol Rhinol (Bord,* 1990, vol. 111 (5), 419-21
- **AVAN, P. ; BÜKI, B. ; MAAT, B. ; DORDAIN, M. ; WIT, H.P.** Middle-ear influence on otoacoustic emissions. I: Noninvasive investigation of the human transmission apparatus and comparison with model results. *Hear.Res.,* 2000, vol. 140, 189-201
- **DE BUKI B ; CHOMICKI A ; DORDAIN M ; LEMAIRE JJ ; WIT HP ; CHAZAL J ; AVAN P.** Middle-ear influence on otoacoustic emissions. II: contributions of posture and intracranial pressure. *Hear Res.,* Février 2000, vol. 140 (1-2), 202-11
- **ZWISLOCKI JJ.** Some current concepts of cochlear mechanics. *Audiology,* 1983, vol. 22 (6), 517-29
- **BÉKÉSY G. VON.** Direct observation of the vibrations of the cochlear partition under a microscope. *Acta Otolaryngol.,* Juin 1952, vol. 42 (3), 197-201